# EUROPÄISCHE PATENTSCHRIFT

(11) **EP 3 651 823 B1**
(45) Veröffentlichungstag und Bekanntmachung des Hinweises auf die Patenterteilung: **20.05.2026**
(21) Anmeldenummer: 18740767.1
(22) Anmeldetag: 06.07.2018
(51) Int. Cl.: A61M 1/14, A61M 60/113, A61M 60/279, A61M 60/37, A61M 60/50, A61M 60/546

(54) **VORRICHTUNGEN ZUM KALIBRIEREN EINER PUMPE FÜR DIE BLUTBEHANDLUNG**
DEVICES FOR CALIBRATING A PUMP FOR BLOOD TREATMENT
DISPOSITIFS D'ÉTALONNAGE D'UNE POMPE DESTINÉE À UN TRAITEMENT DE SANG

(30) Priorität: 10.07.2017 DE 102017115429
(43) Veröffentlichungstag der Anmeldung: 20.05.2020
(73) Patentinhaber: Fresenius Medical Care Deutschland GmbH, 61352 Bad Homburg (DE)
(72) Erfinder: BEDEN, Josef, 55252 Mainz-Kastel (DE); KLEWINGHAUS, Juergen, 61440 Oberursel (DE)
(74) Vertreter: Bobbert & Partner Patentanwälte PartmbB
(86) Internationale Anmeldenummer: PCT/EP2018/068442
(87) Internationale Veröffentlichungsnummer: WO 2019/011822

(56) Entgegenhaltungen:
- EP-A1- 3 031 485
- EP-A1- 3 034 106
- US-A- 5 200 090

## Beschreibung

Die vorliegende Erfindung betrifft eine Steuervorrichtung gemäß Anspruch 1 zum Kalibrieren einer Pumpe für die Blutbehandlung. Zudem betrifft sie eine Blutbehandlungsvorrichtung gemäß Anspruch 12.

Aus der Praxis ist die extrakorporale Blutbehandlung bekannt. Dabei wird dem Patienten Blut entnommen, das entlang eines Blutkreislaufs extrakorporal und z. B. durch einen Blutfilter geführt wird. Hierzu wird i. a. R. eine Blutpumpe verwendet. Da die Genauigkeit der Blutpumpe wie auch anderer Pumpen von Bedeutung sein kann, gilt es, diese zu kalibrieren, wie z.B. in EP 3 034 106 A1, US 5,200,090 oder EP 3 031 485 A1 offenbart.

Die tatsächliche Pumprate kann, insbesondere bei Rollenpumpen, von der vorgegebenen Förderrate abweichen. Die vorgegebene Förderrate kann beispielsweise durch die Drehzahl des Pumpenrotors vorgegeben werden.

Eine Aufgabe der vorliegenden Erfindung ist es, eine Steuervorrichtung anzugeben, mittels welcher ein Verfahren zum Kalibrieren einer Pumpe für die Blutbehandlung durchführbar ist.

Ferner soll eine Blutbehandlungsvorrichtung, mit welcher das Verfahren durchführbar ist, angegeben werden.

Die erfindungsgemäße Aufgabe wird durch eine Steuervorrichtung mit den Merkmalen des Anspruchs 1 gelöst. Zudem wird sie gelöst durch eine Blutbehandlungsvorrichtung mit den Merkmalen des Anspruchs 12.

Alle mit dem hierin offenbarten Verfahren erzielbaren Vorteile lassen sich in bestimmten erfindungsgemäßen Ausführungsformen ungeschmälert auch mit den erfindungsgemäßen Vorrichtungen erzielen.

Die vorliegende Erfindung betrifft ein Verfahren zum Ermitteln einer tatsächlichen Pumprate (kann Volumen pro Zeit sein) und/oder zum Kalibrieren einer Blutpumpe einer Blutbehandlungsvorrichtung, welche mit einem extrakorporalen Blutkreislauf verbunden ist.

Das Verfahren umfasst dabei als ersten von mehreren Schritten das Bereitstellen einer Blutpumpe einer Blutbehandlungsvorrichtung oder das Aufbauen einer Signalverbindung zu einer Blutpumpe. Die Blutpumpe ist mit wenigstens einer ersten Quelle für ein Fluid sowie wenigstens einer ersten Leitung eines extrakorporalen Blutkreislaufs, welche sich stromabwärts an die erste Quelle anschließt, verbunden oder verbindbar, wobei es sich bei dieser ersten Quelle vorzugsweise nicht um einen Patienten und beim Fluid der Quelle vorzugsweise nicht um Blut handelt. Ferner ist die Blutpumpe mit wenigstens einer ersten Aufnahme zum Aufnehmen von Fluiden der ersten Quelle, wobei die Aufnahme mit der ersten Leitung in Fluidverbindung steht, verbindbar oder verbunden. Dabei ist die Aufnahme auf oder an einer ersten Wiegeeinrichtung so angeordnet, dass mittels der Wiegeeinrichtung das Gewicht der Aufnahme und/oder das Gewicht ihres Inhalts bestimmt werden kann.

Das Verfahren umfasst als weiteren Schritt das Einstellen eines Wertes für eine an der Blutpumpe einstellbare Pumprate als eingestellte Pumprate oder das Übermitteln eines solchen Wertes an die Blutpumpe als eingestellte Pumprate.

In einem nächsten Schritt wird die Blutpumpe bei der eingestellten Pumprate während einer gewissen Förderdauer derart betrieben, dass Fluid aus der Quelle als Istvolumen durch die erste Leitung, oder aber Fluid, das in der ersten Leitung vorliegt und durch Fluid aus der Quelle verdrängt wird, in die Aufnahme gefördert wird.

In einem weiteren, vom Verfahren umfassten Schritt, wird ein während einer Förderdauer (einer Zeitspanne) gefördertes Istvolumen mittels der ersten Wiegeeinrichtung anhand einer Änderung des Gewichts der Aufnahme und/oder ihres Inhalts ermittelt. Dabei kann die Dichte des geförderten Fluids berücksichtigt werden. Die Ermittlung des Istvolumens geschieht beispielsweise anhand des Gewichts des tatsächlich geförderten Istvolumens oder anhand einer Gewichtsänderung der Aufnahme oder ihres Inhalts aufgrund des geförderten Volumens, oder hiervon ausgehend oder hierauf basierend, etwa durch bekannte Beziehungen zwischen Volumen und Gewicht des geförderten Fluids.

Das Verfahren umfasst als weiteren Schritt das Ermitteln einer mathematischen Beziehung zwischen dem Istvolumen und einem Sollvolumen, welches sich aus der Förderdauer und der eingestellten Pumprate ergibt und/oder das Ermitteln der tatsächlichen Pumprate aus dem Istvolumen und dem Sollvolumen.

Die erfindungsgemäße Steuer- oder Regelvorrichtung ist geeignet und vorgesehen und/oder ausgelegt und/oder konfiguriert zur Durchführung des Verfahrens.

Die vorliegende Erfindung betrifft zudem eine Blutbehandlungsvorrichtung. Die erfindungsgemäße Blutbehandlungsvorrichtung weist einen extrakorporalen Blutkreislauf auf oder ist hiermit verbunden. Die Blutbehandlungsvorrichtung weist weiter auf oder ist verbunden mit wenigstens einer Blutpumpe (sie dient während einer Blutbehandlung dem Fördern von Blut), welche mit wenigstens einer ersten Quelle für ein Fluid und mit wenigstens einer ersten Leitung, welche sich stromabwärts an die erste Quelle anschließt, verbunden ist. Ferner weist die Blutbehandlungsvorrichtung auf oder ist verbunden mit wenigstens einer Aufnahme zum Aufnehmen von Fluid der ersten Quelle, wobei die Aufnahme mit der ersten Leitung in Fluidverbindung steht, sowie mit einer ersten Wiegeeinrichtung für die Aufnahme.

Die erfindungsgemäße Blutbehandlungsvorrichtung weist zudem auf oder ist verbunden mit einer Steuer- oder Regelvorrichtung, welche zum Einstellen eines Wertes für eine an der Blutpumpe einstellbare Pumprate als durch den Nutzer eingestellte Pumprate konfiguriert ist. Ferner ist die Steuer- oder Regelvorrichtung konfiguriert, um wenigstens eine Blutpumpe bei der eingestellten Pumprate während einer gewissen Förderdauer derart zu betreiben, dass Fluid aus der Quelle durch die erste Leitung in die Aufnahme gefördert wird. Des Weiteren ist die Steuer- oder Regelvorrichtung, welche mit der Blutbehandlungsvorrichtung verbunden ist, konfiguriert zum Ermitteln eines während der Förderdauer geförderten Istvolumens und/oder einer tatsächlichen Pumprate mittels der ersten Wiegeeinrichtung. Sie ist außerdem konfiguriert, um eine mathematische Beziehung zwischen dem Istvolumen und dem Sollvolumen zu ermitteln, welches sich aus der Förderdauer und der eingestellten Pumprate ergibt.

Die erfindungsgemäße Blutbehandlungsvorrichtung ist vorgesehen und ausgestaltet und/oder ausgestattet zur Durchführung des Verfahrens.

Die erfindungsgemäße Steuervorrichtung ist geeignet und vorgesehen und/oder ausgelegt und/oder konfiguriert zur Durchführung des Verfahrens im Zusammenwirken mit den hierzu jeweils erforderlichen Einrichtungen, beispielsweise wie hierin beschrieben.

Erfindungsgemäße Ausführungsformen können manche, einige oder alle der folgenden Merkmale in beliebiger Kombination aufweisen, soweit dies für den Fachmann nicht erkennbar technisch unmöglich ist. Vorteilhafte Weiterbildungen der vorliegenden Erfindung sind jeweils auch Gegenstand der Unteransprüche.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von einer Ausführungsform die Rede ist, so stellt dies eine erfindungsgemäße, beispielhafte Ausführungsform dar.

In bestimmten Ausführungsformen ist die Blutbehandlungsvorrichtung eine Hämodialysevorrichtung, Hämofiltrationsvorrichtung oder Hämodiafiltrationsvorrichtung, insbesondere eine Vorrichtung für die chronische Nierenersatztherapie oder für die kontinuierliche Nierenersatztherapie (CRRT = continuous renal replacement therapy).

In manchen Ausführungsformen weist die Blutbehandlungsvorrichtung eine, vorzugsweise erfindungsgemäße, Steuer- oder Regelvorrichtung auf. Die Steuer- oder Regelvorrichtung kann programmiert und/oder konfiguriert sein, um das Verfahren im Zusammenwirken mit weiteren Einrichtungen, insbesondere einer Blutbehandlungsvorrichtung, auszuführen.

Der extrakorporale Blutkreislauf ist in bestimmten Ausführungsformen der vorliegenden Erfindung ein Schlauchset. In jedem Fall ist der extrakorporale Blutkreislauf vorgesehen zum extrakorporalen Leiten von Blut eines Patienten, beispielsweise bei der Hämodialyse, der Hämofiltration, der Hämodiafiltration oder dergleichen.

In einigen erfindungsgemäßen Ausführungsformen ist der extrakorporale Blutkreislauf zumindest abschnittsweise als integraler und ggf. unlösbarer Bestandteil einer Blutkassette ausgestaltet, in anderen nicht. So kann sich ein frei beweglicher Schlauchabschnitt des extrakorporalen Blutkreislaufs einstückig oder integral auf oder in der Funktionseinrichtung, beispielsweise einer Blutkassette, fortsetzen und umgekehrt.

Eine Blutkassette ist in bestimmten Ausführungsformen der vorliegenden Erfindung eine Einrichtung, welche bei einer Blutbehandlung verwendet wird. Beispiele für Blutkassetten schließen Disposables oder Einmal-Blutkassetten ein.

Beispielhafte Ausführungsformen einer Blutkassette sind insbesondere in der Anmeldung der Anmelderin mit der Veröffentlichungsnummer DE 10 2009 018 664 A1 mit dem Titel "Externe Funktionseinrichtung, Blutbehandlungsvorrichtung zum Aufnehmen einer erfindungsgemäßen externen Funktionseinrichtung, sowie Verfahren", die am 23.04.2009 beim Deutschen Patent- und Markenamt eingereicht wurde, und in der Anmeldung der Anmelderin mit der Veröffentlichungsnummer DE 10 2009 024 468 A1 mit demselben Titel, die am 10.06.2009 beim Deutschen Patent- und Markenamt eingereicht wurde, offenbart.

Der arterielle Leitungsabschnitt des extrakorporalen Blutkreislaufs ist in bestimmten Ausführungsformen der vorliegenden Erfindung jener Leitungsabschnitt, in welchen das den Patientenkörper zur extrakorporalen Blutbehandlung verlassende Patientenblut einströmt und in welchem es sich vor Eintritt in die Blutbehandlungseinrichtung, z. B. einen Dialysator, befindet.

In bestimmten Ausführungsformen der vorliegenden Erfindung ist oder umfasst der erste Abschnitt des arteriellen Leitungsabschnitts den arteriellen Nadelanschluss zum Patienten, z. B. den arteriellen Nadelanschluss bei einem Double-Needle-Dialyseverfahren.

In manchen Ausführungsformen umfasst das Verfahren einen Schritt, welcher ein Verhältnis zwischen der eingestellten Pumprate und der tatsächlichen Pumprate ermittelt. Dabei wird die tatsächliche Pumprate unter Berücksichtigung des Istvolumens und der Förderdauer ermittelt.

In einigen Ausführungsformen wird während oder mittels des Verfahrens ein Korrekturfaktor oder ein Kalibrierfaktor für die Blutpumpe festgelegt. Dieser wird anhand des Verhältnisses zwischen der an der Blutpumpe eingestellten Pumprate und der tatsächlichen Pumprate und/oder anhand des Istvolumens und des Sollvolumens festgelegt, berechnet oder bestimmt.

Erfindungsgemäß wird das Erzeugen von gleichen Druckverhältnissen vor und nach der Förderung (oder Förderdauer) in der ersten Leitung als weiterer Schritt des Verfahrens angesehen. Die Bezeichnung "nach der Förderung" kann dabei einen Zeitpunkt vor dem Ermitteln des Istvolumens und/oder der tatsächlichen Pumprate beschreiben. In manchen Ausführungsformen umfasst das Verfahren das Erzeugen von gleichen Druckverhältnissen während der Förderdauer in der ersten Leitung.

Unter "gleichen Druckverhältnissen während der Förderung" kann z. B. verstanden werden, dass zwei, drei oder mehr Messungen, die während der Förderdauer gemacht werden, zu gleichen Ergebnissen oder zu Ergebnissen, die als gleich gelten (da sie z. B. auf Messschwankungen usw. zurückzuführen sind), führen. Alternativ kann hierunter verstanden werden, dass der gemessene Druck sich zumindest über einen Zeitabschnitt der Förderdauer oder einen Zeitraum innerhalb der Förderdauer, oder über die gesamte Förderdauer, nicht ändert.

In einigen Ausführungsformen umfasst der extrakorporale Blutkreislauf einen Blutfilter oder einen Dialysator, welcher eine zumeist semi-permeable Membran aufweist, oder ist hiermit verbunden.

Die Aufnahme steht in Fluidverbindung mit der ersten Leitung.

In einigen Ausführungsformen steht die Aufnahme stromab des Blutfilters oder Dialysators und durch dessen Membran hindurch in Fluidverbindung mit der ersten Leitung.

In einigen Ausführungsformen des Verfahrens ist der Dialysator mit einer Dialysierflüssigkeitszuleitung und mit einer Dialysatleitung verbunden. In der Dialysatleitung kann eine Effluentpumpe angeordnet sein. Dabei ist ein Rückschlagventil und/oder eine okkludierende Pumpe in die Dialysierflüssigkeitszuleitung integriert. Das Rückschlagventil dient vorzuweise zum Verhindern eines ungewünschten Flusses in Richtung entgegen der Strömungsrichtung der Dialysierflüssigkeitszuleitung im bestimmungsgemäßen Gebrauch und/oder in Richtung weg vom Blutfilter.

In manchen Ausführungsformen steht der Dialysator mit einer arteriellen Patientenleitung und mit einer venösen Patientenleitung in Fluidverbindung, wobei in oder an der venösen Patientenleitung eine Schlauchklemme angeordnet ist. Dabei umfasst das Verfahren ferner ein Schließen der Schlauchklemme an der venösen Patientenleitung während der Förderdauer und/oder der Durchführung des Verfahrens.

In einigen Ausführungsformen des Verfahrens fördern die Blutpumpe und eine Effluentpumpe während der Förderdauer mit derselben Pumprate.

Die Effluentpumpe kann eine Pumpe zum Abführen verbrauchten Dialysats aus dem Blutfilter oder eine Filtratpumpe sein.

In einigen Ausführungsformen umfasst das Verfahren in einem Schritt das Regeln der Pumpe auf gleichbleibenden Druck in der ersten Leitung während der Förderdauer.

In gewissen Ausführungsformen wird die Effluentpumpe zum Erzeugen von gleichen Druckverhältnissen gedreht, insbesondere durch die Steuer- oder Regelvorrichtung, insbesondere druckgesteuert.

In manchen Ausführungsformen ist das erste Fluid eine Kochsalzlösung, eine Dialysierflüssigkeit oder ein Substituat, insbesondere eine Primingflüssigkeit.

In einigen Ausführungsformen umfasst das Verfahren den Schritt der Bereitstellung der Blutbehandlungsvorrichtung als Hämodialysevorrichtung, Hämofiltrationsvorrichtung oder Hämodiafiltrationsvorrichtung, insbesondere als eine Vorrichtung für die chronische Nierenersatztherapie oder für die kontinuierliche Nierenersatztherapie (CRRT = continuous renal replacement therapy).

Der venöse Leitungsabschnitt des extrakorporalen Blutkreislaufs ist in manchen Ausführungsformen der vorliegenden Erfindung jener Leitungsabschnitt, aus welchem das extrakorporal behandelte Patientenblut nach seiner Behandlung in einer Blutbehandlungseinrichtung, beispielsweise einem Dialysator, zum Patientenkörper hin oder in diesen zurück strömt.

Die erfindungsgemäße Steuervorrichtung ist in einigen erfindungsgemäßen Ausführungsformen als Regelvorrichtung ausgestaltet.

Die erfindungsgemäße Blutbehandlungsvorrichtung weist in bestimmten erfindungsgemäßen Ausführungsformen wenigstens eine erfindungsgemäße Steuervorrichtung auf.

Vorzugsweise läuft das Verfahren nicht während einer Patientenbehandlung ab. Vorzugsweise läuft das Verfahren ab, ohne dass ein Patient mit der Blutbehandlungsvorrichtung verbunden ist. Vorzugsweise läuft das Verfahren ab, ohne dass dem Patienten Blut entnommen wurde oder wird und/oder vorzugsweise ohne Blutrückgabe an den Patienten.

Als Verfahren zum Umsetzen eines eingestellten Wertes für eine an der Blutpumpe einstellbare Pumprate als eingestellte Pumprate kommen z. B. Verfahren in Betracht, welche sich auf die Anzahl der Pumpbewegungen der Blutpumpen z. B. die Anzahl der Umdrehungen ihres Rotors, stützen.

Manche oder alle erfindungsgemäßen Ausführungsformen können einen, mehrere oder alle der oben und/oder im Folgenden genannten Vorteile aufweisen.

Ein mittels mancher Ausführungsformen der vorliegenden Erfindung erzielbarer Vorteil besteht darin, dass die Blutförderrate durch die Blutpumpe genau in Erfahrung gebracht werden kann. Dies kann insbesondere für die Behandlung des Patienten von Belang sein, in welchen in Abhängigkeit der Blutförderrate Medikamente zudosiert werden, etwa bei der regionalen Antikoagulation, bei welcher Citrat in bestimmten Verhältnissen in Abhängigkeit des Blutstroms in das strömende Blut zugegeben werden. Die genaue Kenntnis der Blutförderrate ist hier von besonderem Vorteil. Dasselbe gilt für Fälle, in welchen nur geringe Blutflüsse mittels der Blutpumpe erzeugt werden, wie etwa bei pädiatrischen Anwendungen. Hier gilt es, die Blutförderrate besonders genau zu kennen.

Ein weiterer Vorteil kann darin bestehen, auf das Vorsehen und das Verwenden von Flusssensoren zur Bestimmung des genauen Blutflusses verzichten zu können. Der genaue Blutfluss ist bei Kenntnis der genauen Blutförderrate der Blutpumpe - jedenfalls ausreichend genau - bekannt.

Ein wiederum weiterer Vorteil kann darin bestehen, dass in manchen erfindungsgemäßen Ausführungsformen zum Wiegen der Aufnahme zum Ermitteln des Istvolumens solche Wiegeeinrichtungen verwendet werden, die ohnehin vorhanden sind, etwa weil sie bei einer sich nach Abschluss im Rahmen des erfindungsgemäßen Verfahrens durchgeführten Blutbehandlung zur Bilanzierung verwendet werden, insbesondere bei der kontinuierlichen Nierenersatztherapie (CRRT).

Dabei kann ein besonderer Vorteil darin bestehen, dass die Blutpumpe automatisch kalibriert und/oder der Korrekturfaktor automatisch ermittelt werden kann. Es bedarf hierzu keines Umlagerns oder Anordnens von Fluidbehältern, beispielsweise so, dass diese auf Waagen zu liegen kommen, kein Vorsehen von eigens zum Kalibrieren vorgesehenen Fluidbehältern, usw. Damit kann in bestimmten Ausführungsformen Arbeit und Zeit eingespart werden. Es kann vorteilhaft auf Anordnungen zurückgegriffen werden, welche ohne Änderungen bei der folgenden Blutbehandlung verwendet werden.

Im Folgenden wird die vorliegende Erfindung unter Bezugnahme auf die beigefügten Figuren rein exemplarisch beschrieben. In ihnen bezeichnen selbe Bezugszeichen gleiche oder selbe Komponenten. Es gilt:
- **Fig. 1**: zeigt in vereinfachter Darstellung eine erfindungsgemäße Blutbehandlungsvorrichtung mit einem extrakorporalen Blutkreislauf in einer ersten Ausführungsform;
- **Fig. 2**: zeigt in vereinfachter Darstellung eine erfindungsgemäße Blutbehandlungsvorrichtung mit einem extrakorporalen Blutkreislauf in einer zweiten Ausführungsform; und
- **Fig. 3**: zeigt in vereinfachter Darstellung eine erfindungsgemäße Blutbehandlungsvorrichtung mit einem extrakorporalen Blutkreislauf in einer dritten Ausführungsform.

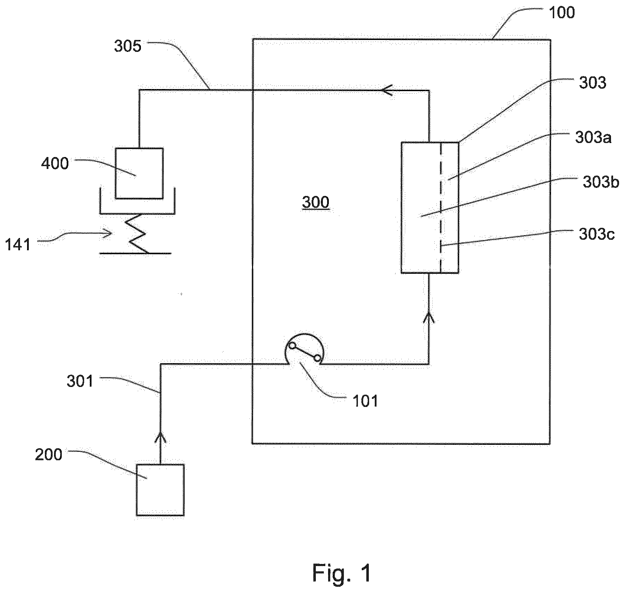

**Fig. 1** zeigt in vereinfachter Darstellung eine erfindungsgemäße Blutbehandlungsvorrichtung 100, verbunden mit einem extrakorporalen Blutkreislauf 300, während der Ausführung des hierin beschriebenen Verfahrens. Fig. 1 zeigt das Grundprinzip der vorliegenden Erfindung.

Der extrakorporale Blutkreislauf 300 weist eine erste Leitung 301, hier in Form eines arteriellen Leitungsabschnitts, auf.

Die erste Leitung 301 steht in Fluidverbindung mit einer Blutbehandlungseinrichtung, hier exemplarisch ein Blutfilter 303 oder Dialysator. Der Blutfilter 303 weist eine Dialysierflüssigkeitskammer 303a und eine Blutkammer 303b auf, welche durch eine zumeist semi-permeable Membran 303c voneinander getrennt sind.

Der extrakorporale Blutkreislauf 300 weist ferner wenigstens eine zweite Leitung 305, hier in Form eines venösen Leitungsabschnitts, auf. Sowohl die erste Leitung 301 als auch die zweite Leitung 305 können zur Verbindung mit dem Gefäßsystem des nicht dargestellten Patienten dienen.

Die in Fig. 1 nur durch einige ihrer Einrichtungen repräsentierte Blutbehandlungsvorrichtung 100, mittels welcher das hier beschriebene Verfahren abläuft, weist eine Blutpumpe 101 auf. Die Blutpumpe 101 fördert bei der Behandlung des Patienten Blut durch Abschnitte des extrakorporalen Blutkreislaufs 300 und in Richtung zum Blutfilter 303, wie die kleinen Pfeilspitzen, welche in den Figuren allgemein die Strömungsrichtung angeben, zeigen.

Zum Kalibrieren der Blutpumpe 101 wird mittels der Blutpumpe 101, die optional als Rollenpumpe oder als anderweitig okkludierende Pumpe ausgestaltet sein kann, Fluid aus einer Quelle 200 entlang der ersten Leitung 301 in Richtung der Aufnahme 400 gepumpt.

Die Quelle 200 kann beispielsweise ein Beutel oder ein Container sein. Dasselbe gilt für die Aufnahme 400. Die Quelle 200 kann ferner eine Fluidleitung sein, aus der online und/oder kontinuierlich erzeugte oder gemischte Flüssigkeit bereitgestellt wird, z. B. ein Hydraulikausgang oder -anschluss der Blutbehandlungsvorrichtung.

Die Aufnahme 400 ist mit einer ersten Wiegeeinrichtung 141 zum Wiegen ihres Gewichts oder des in ihr aufgenommenen Fluids oder zum Bestimmen einer Gewichtsänderung verbunden. Beispielsweise kann die Aufnahme 400 als Auffangbeutel auf einer Wiegefläche der ersten Wiegeeinrichtung 141 liegen oder an einem Wiegehaken hängen.

Fördert die Blutpumpe 101 nun über einen vorbestimmten oder bestimmbaren Zeitraum, nämlich die Förderdauer T, so lässt sich anhand der an der Blutpumpe 101 - vom Nutzer oder von einem Testprogramm - eingestellten Pumprate P1 und der Förderdauer T anhand der einfachen Beziehung P1*T = VS jenes Volumen an Fluid ermitteln, das gefördert worden wäre, würde die Blutpumpe 101 tatsächlich mit der eingestellten Pumprate P1 fördern. Dieses Volumen wird hierin als Sollvolumen VS bezeichnet.

In der Praxis kann das tatsächliche Fördervolumen, das hierin als Istvolumen VI bezeichnet wird, vom Sollvolumen abweichen.

Das Istvolumen VI findet sich nach Ablauf der Förderdauer T jedoch in der Aufnahme 400 wieder. Es lässt sich anhand der Gewichtsmessung, die hier exemplarisch mittels der ersten Wiegeeinrichtung 141 erfolgt, bestimmen; sein Gewicht entspricht der Zunahme des Gewichts der Aufnahme 400 zwischen einer Messung, die vor Beginn der Förderdauer T liegt, und einer Messung zu einem Zeitpunkt, der nach der Förderdauer T liegt.

Ein Vergleich des gemessenen Istvolumens VI mit dem errechneten Sollvolumen VS kann zum Berechnen eines Korrekturfaktors oder eines Kalibrierwerts der eingestellten Pumprate oder der Blutpumpe 101 dienen.

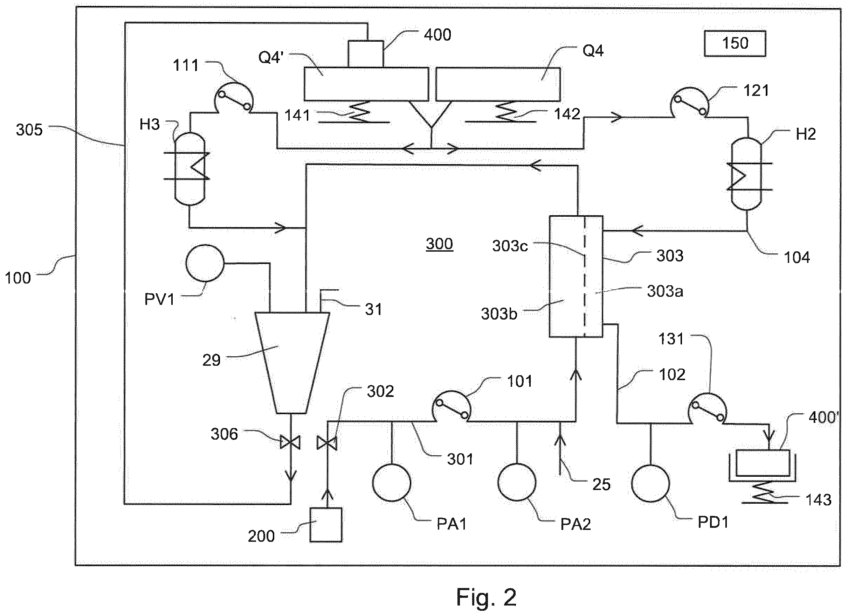

Eine erst zu Fig. 2 beschriebene und dort gezeigt Steuer-oder Regelvorrichtung 150 (siehe Fig. 2) kann dazu konfiguriert sein, das vorstehende Verfahren auszuführen. Optional erfolgt eine automatische Ausführung. Optional startet die Ausführung automatisch, etwa im Rahmen einer Testfunktion, eines Selbstchecks beim Starten der Blutbehandlungsvorrichtung, während oder im Anschluss an einen Primingvorgang, usw.

**Fig. 2** zeigt in vereinfachter Darstellung eine erfindungsgemäße Blutbehandlungsvorrichtung 100 mit einem extrakorporalen Blutkreislauf 300 in einer zweiten Ausführungsform.

Neben der vorgenannten Blutpumpe 101 weist die in Fig. 2 gezeigte Anordnung ferner rein optional eine Reihe weiterer, jeweils optionaler Pumpen, nämlich die Pumpe 111 für Substituat, die Pumpe 121 für Dialysierflüssigkeit, und die Pumpe 131 für Dialysat und/oder Effluent auf.

Die Pumpe 121 ist vorgesehen, um Dialysierflüssigkeit aus einer Quelle Q4, beispielsweise einem Beutel, heraus und über eine optional vorhandene Beutelheizung mit einem Beutel H2 mittels einer Dialysierflüssigkeitszuleitung 104 zuzuführen.

Die auf diese Weise zugeführte Dialysierflüssigkeit tritt über eine Dialysatleitung 102, unterstützt durch die Pumpe 131, wieder aus und kann verworfen werden.

Stromauf der Blutpumpe 101 ist ein optionaler arterieller Sensor PA1 vorgesehen. Während einer Behandlung des Patienten misst er den Druck in der arteriellen Leitung.

Stromab der Blutpumpe 101, jedoch stromauf des Blutfilters 303 und, falls vorgesehen, einer Zugabestelle 25 für Heparin, ist ein weiterer, optionaler Drucksensor PA2 vorgesehen. Er misst den Druck stromauf des Blutfilters 303 ("prä-Hämofilter").

Ein wiederum weiterer Drucksensor kann als PD1 stromab des Blutfilters 303, jedoch vorzugsweise stromauf der Pumpe 131 in der Dialysatleitung 102 zum Messen eines Filtratdrucks stromabwärts des Blutfilters 303 vorgesehen sein.

Blut, das den Blutfilter 303 verlässt, durchströmt eine optionale venöse Blutkammer 29, welche eine optional verschließbare Entlüftungseinrichtung 31 aufweisen und mit einem weiteren Drucksensor PV1 in Fluidverbindung stehen kann.

Im Beispiel der Fig. 2 unterliegen die Quelle Q4 und eine optionale weitere Quelle Q4', aus welcher mittels der Pumpe 111 über eine weitere Beutelheizung mit Beutel H3 Substituat entnommen wird, sowie das aufgefangene oder verworfene Dialysat optional einer Bilanzierung. Zum Zweck der Bilanzierung sind neben der aus Fig. 1 bekannten ersten Wiegeeinrichtung 141 optional zwei weitere Waagen oder Wiegeeinrichtungen 142 und 143 vorgesehen.

Die hier exemplarisch gezeigte Bilanzierung entspricht einer gravimetrischen Bilanzierung. Die vorliegende Erfindung umfasst aber auch jede andere Bilanzierung, beispielsweise mittels Bilanzkammern.

Die in Fig. 2 gezeigte exemplarische Anordnung weist eine Steuer- oder Regelvorrichtung 150 auf. Sie kann mit jeder der hierin genannten Komponenten - jedenfalls oder insbesondere mit der Blutpumpe 101 - in kabelgebundener oder kabelloser Signalverbindung zur Steuerung oder Regelung der Blutbehandlungsvorrichtung 100 stehen. Sie ist optional konfiguriert, um das hierin beschriebene Verfahren auszuführen.

Die erste Leitung 301 ist optional mit einer Schlauchklemme 302 zum Sperren oder Schließen der Leitung 301 verbunden. Die zweite Leitung 305 ist optional mit einer Schlauchklemme 306 zum Sperren oder Schließen der Leitung 305 verbunden.

Zwar ist die Ausführungsform der Fig. 2 mit einer Aufnahme 400 für Fluid gezeigt, deren Gewicht von der ersten Wiegeeinrichtung 141 ermittelt wird, wobei die Aufnahme 400 der Blutseite zugeordnet werden kann aufgrund ihrer Fluidverbindung mit dem Blutkreislauf, hier stromab des Blutfilters 303.

Allerdings kann die Aufnahme alternativ auf der Hydraulikseite oder Filtratseite (d. h. nicht auf der Blutseite sondern z. B. im Bereich der Komponenten 102 und 131) angeordnet sein. So kann die Aufnahme mittels der dritten Wiegeeinrichtung 143 gewogen werden, wie dies mit dem Bezugszeichen 400' angedeutet ist.

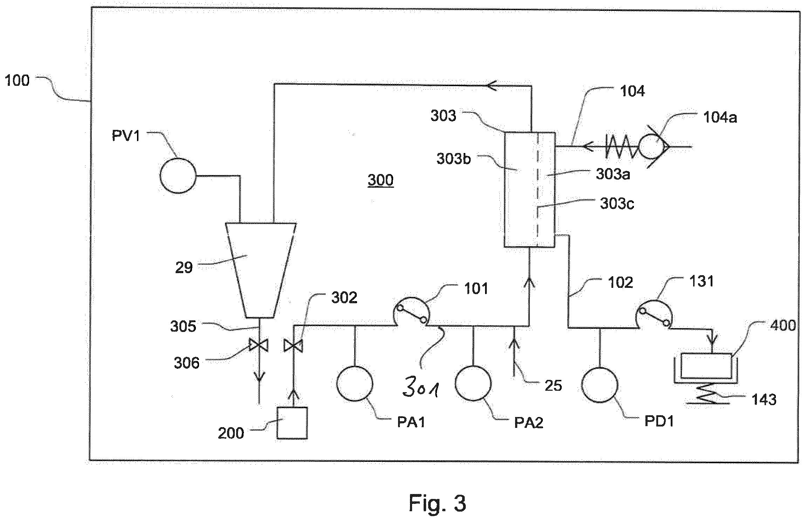

**Fig. 3** zeigt in vereinfachter Darstellung eine erfindungsgemäße Blutbehandlungsvorrichtung 100 mit einem extrakorporalen Blutkreislauf 300 in einer dritten Ausführungsform.

Die Darstellung der Fig. 3 unterscheidet sich von jener der Fig. 2 bereits dadurch, dass eine Reihe von Komponenten, die in Fig. 2 gezeigt sind, der übersichtlichen Darstellung wegen, in Fig. 3 nicht erneut gezeigt sind. Sie können optional jedoch auch hier vorhanden sein.

Die in Fig. 3 gezeigte Ausführungsform unterscheidet sich von der in Fig. 2 gezeigten jedenfalls darin, dass in der Dialysierflüssigkeitszuleitung 104 ein optionales Rückschlagventil 104a angeordnet ist. Das Rückschlagventil 104a öffnet sich bei entsprechend hohem Druck, um einen Fluss durch die Dialysierflüssigkeitszuleitung 104 in Richtung zum Blutfilter 303 zu erlauben. Einen Fluss in entgegengesetzter Richtung durch die Dialysierflüssigkeitszuleitung 104 hindurch verhindert das Rückschlagventil 104a jedoch.

In der Ausführungsform der Fig. 3 fördert die Blutpumpe 101 Fluid aus der Quelle 200 in den Blutfilter 303. Da die Schlauchklemme 306 der zweiten Leitung 305 verschlossen ist, tritt das geförderte Fluid über die Membran 303c von der Blutkammer 303b in die Dialysierflüssigkeitskammer 303a über. Das Rückschlagventil 104a - oder eine andere okkludierende Vorrichtung, etwa eine Rollenpumpe anstelle des Rückschlagventils oder ergänzend zum Rückschlagventil - verhindert, dass das in die Dialysierflüssigkeitskammer 303a übergetretene Fluid letztere auf anderem Weg verlässt als über die Leitung 102. Die Förderung des Fluids entlang des vorstehend beschriebenen Wegs wird durch Pumptätigkeit der in die Dialysatleitung 102 eingefügten Pumpe 131, der Effluentpumpe, gewährleistet. Schließlich wird das Fluid in die Aufnahme 400 geleitet. Deren Gewichtsänderung kann mittels der dritten Wiegeeinrichtung 143 ermittelt werden. Aus ihr ergibt sich das Istvolumen VI, welches die Blutpumpe 101 während der Förderdauer T gefördert hat.

Folgende Merkmale können, obgleich in den Figuren nicht gezeigt, in jeder erfindungsgemäßen Ausführungsform wiederum rein optional und in beliebiger Kombination vorgesehen sein:
Die erste Leitung 301 kann ein arterielles Septum, optional in Gestalt einer Zugabeeinrichtung, aufweisen.

Die erste Leitung 301 und/oder die zweite Leitung 305 können einen Luftblasendetektor/optischen Sensor aufweisen.

Der Blutkreislauf kann zumindest teilweise Teil einer Blutkassette, welche ein Hartteil aufweist, ganz oder teilweise bedeckt durch eine Folie, sein oder hiermit verbunden sein.

### Bezugszeichenliste

- 25: Zugabestelle für Heparin (optional)
- 29: venöse Blutkammer (optional)
- 31: Entlüftungseinrichtung
- 100: Blutbehandlungsvorrichtung
- 101: Blutpumpe
- 102: Dialysatleitung
- 104: Dialysierflüssigkeitszuleitung
- 104a: Rückschlagventil
- 111: Pumpe für Substituat
- 121: Pumpe für Dialysierflüssigkeit
- 131: Pumpe für Dialysat und/oder Effluent
- 141: erste Wiegeeinrichtung
- 142: zweite Wiegeeinrichtung
- 143: dritte Wiegeeinrichtung
- 150: Steuer- oder Regelvorrichtung
- 200: Quelle von Fluid
- 300: extrakorporaler Blutkreislauf
- 301: erste Leitung (arterieller Leitungsabschnitt)
- 302: Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: Schlauchklemme
- 400: Aufnahme für Fluid
- 400': Aufnahme für Fluid
- H2: Beutelheizung mit Beutel (Dialysierflüssigkeit)
- H3: Beutelheizung mit Beutel (Substituat)
- PA1, PA2: arterieller Drucksensor (optional)
- PD1: Drucksensor zum Messen des Filterdrucks
- PV1: Drucksensor (optional)
- P1: eingestellte Pumprate
- Q4: Quelle mit Dialysierflüssigkeit
- Q4': Quelle (Substituat), optional
- T: Förderdauer
- VI: Istvolumen
- VS: Sollvolumen

## Patentansprüche

1. Steuer- oder Regelvorrichtung (150), geeignet und vorgesehen und/oder ausgelegt und/oder konfiguriert zur Durchführung eines Verfahrens zum Ermitteln einer tatsächlichen Pumprate und/oder zum Kalibrieren einer Blutpumpe (101) einer Blutbehandlungsvorrichtung (100), welche mit einem extrakorporalen Blutkreislauf (300) mit einem Dialysator (303), welcher eine semi-permeable Membran (303c) aufweist, verbunden ist, mit den Schritten:
- Aufbauen einer Signalverbindung zu der Blutpumpe (101), welche aufweist oder verbindbar oder verbunden ist mit:
- wenigstens eine(r) erste(n) Quelle (200) für ein Fluid;
- wenigstens eine(r) erste(n) Leitung (301) eines extrakorporalen Blutkreislaufs (300), welche sich stromabwärts an die erste Quelle (200) anschließt;
- wenigstens eine(r) Aufnahme (400) zur Aufnahme von Fluid der ersten Quelle (200), wobei die Aufnahme (400) in Fluidverbindung steht mit der ersten Leitung (301);
- wobei die Aufnahme (400) auf oder an einer ersten Wiegeeinrichtung (141) derart angeordnet ist, dass mittels der Wiegeeinrichtung (141) das Gewicht der Aufnahme (400) und/oder ihres Inhalts bestimmbar ist;
wobei das Verfahren die weiteren Schritte umfasst:
- Einstellen eines Wertes für eine an der Blutpumpe (101) einstellbare Pumprate als eingestellte Pumprate (P1), oder Übermitteln eines solchen Wertes an die Blutpumpe (101) als eingestellte Pumprate (P1);
- Betreiben der Blutpumpe (101) bei der eingestellten Pumprate (P1) während einer Förderdauer (T) derart, dass Fluid aus der ersten Quelle (200) als Istvolumen (VI) durch die erste Leitung (301) in die Aufnahme (400) gefördert wird;
- Ermitteln eines während der Förderdauer (T) geförderten Istvolumens (VI) mittels der ersten Wiegeeinrichtung (141) anhand einer Änderung des Gewichts der Aufnahme (400) oder ihres Inhalts;
- Ermitteln einer mathematischen Beziehung zwischen dem Istvolumen (VI) und einem sich aus der Förderdauer (T) und der eingestellten Pumprate (P1) ergebenden Sollvolumen (VS),
- Erzeugen von gleichen Druckverhältnissen vor und nach der Förderdauer (T) in der ersten Leitung (301).

2. Steuer- oder Regelvorrichtung (150), nach Anspruch 1, wobei das Verfahren den Schritt umfasst:
- Ermitteln eines Verhältnisses zwischen der eingestellten Pumprate (P1) und einer tatsächlichen Pumprate, wobei die tatsächliche Pumprate unter Berücksichtigung des Istvolumens (VI) und der Förderdauer (T) ermittelt wird.

3. Steuer- oder Regelvorrichtung (150), nach Anspruch 1 oder 2, wobei das Verfahren den Schritt umfasst:
- Festlegen eines Korrekturfaktors oder Kalibrierfaktors für die Blutpumpe (101) anhand des Verhältnisses zwischen der an der Blutpumpe (101) eingestellten Pumprate (P1) und der tatsächlichen Pumprate und/oder anhand des Istvolumens (VI) und des Sollvolumens (VS).

4. Steuer- oder Regelvorrichtung (150), nach einem der vorangegangenen Ansprüche, wobei das Verfahren den Schritt umfasst:
- Erzeugen von gleichen Druckverhältnissen während der Förderdauer (T) in der ersten Leitung (301).

5. Steuer- oder Regelvorrichtung (150), nach einem der vorangegangenen Ansprüche, wobei die Aufnahme (400) stromab des Dialysators (303) in Fluidverbindung mit der ersten Leitung (301) steht.

6. Steuer- oder Regelvorrichtung (150), nach einem der vorangegangenen Ansprüche, wobei die Aufnahme (400) stromab des Dialysators (303) und durch dessen Membran (303c) hindurch in Fluidverbindung mit der ersten Leitung (301) steht.

7. Steuer- oder Regelvorrichtung (150), nach einem der vorangegangenen Ansprüche, wobei der Dialysator (303) mit einer Dialysierflüssigkeitszuleitung (104) und einer Dialysatleitung (102) verbunden ist, wobei in die Dialysierflüssigkeitszuleitung (104) ein Rückschlagventil (104a) zum Verhindern eines ungewünschten Flusses, insbesondere in Richtung entgegen der Strömungsrichtung der Dialysierflüssigkeitszuleitung (104) und/oder in Richtung weg vom Dialysator (303) und/oder eine okkludierende Pumpe integriert ist.

8. Steuer- oder Regelvorrichtung (150), nach einem der vorangegangenen Ansprüche, wobei der Dialysator (303) mit einer arteriellen Patientenleitung (301) und mit einer venösen Patientenleitung (305) in Fluidverbindung steht, wobei in oder an der venösen Patientenleitung (305) eine Schlauchklemme (306) angeordnet ist, wobei das Verfahren ferner ein Schließen der Schlauchklemme (306) an der venösen Patientenleitung (305) während der Förderdauer (T) umfasst.

9. Steuer- oder Regelvorrichtung (150), nach einem der vorangegangenen Ansprüche, wobei die Blutpumpe (101) und eine Effluentpumpe (131) während der Förderdauer (T) mit derselben Pumprate fördern.

10. Steuer- oder Regelvorrichtung (150), nach Anspruch 10, wobei zum Erzeugen von gleichen Druckverhältnissen die Effluentpumpe (131), durch die Steuer- oder Regelvorrichtung (150), insbesondere druckgesteuert, gedreht wird.

11. Steuer- oder Regelvorrichtung (150), nach einem der vorangegangenen Ansprüche, mit dem Schritt:
- Bereitstellen der Blutbehandlungsvorrichtung (100) als Hämodialysevorrichtung, Hämofiltrationsvorrichtung oder Hämodiafiltrationsvorrichtung, insbesondere ausgestaltet als Vorrichtung für die chronische Nierenersatztherapie oder für die kontinuierliche Nierenersatztherapie (CRRT = continuous renal replacement therapy).

12. Blutbehandlungsvorrichtung (100), welche einen extrakorporalen Blutkreislauf (300) aufweist oder hiermit verbunden ist, wobei die Blutbehandlungsvorrichtung (100) weiter aufweist oder verbunden ist mit:
- einer Blutpumpe (101), welche verbindbar oder verbunden ist mit:
- wenigstens einer ersten Quelle (200) für ein Fluid;
- wenigstens einer ersten Leitung (301), welche sich stromabwärts an die erste Quelle (200) anschließt;
- wenigstens einer Aufnahme (400) zur Aufnahme von Fluid der ersten Quelle (200), wobei die Aufnahme (400) in Fluidverbindung steht mit der ersten Leitung (301);
- einer ersten Wiegeeinrichtung (141) für die Aufnahme (400);
einer Steuer- oder Regelvorrichtung (150) gemäß einem der Ansprüche 1-11 und/oder konfiguriert zum:
- Einstellen eines Wertes für eine an der Blutpumpe (101) einstellbare Pumprate als eingestellte Pumprate (P1) durch den Nutzer oder automatisch durch die Steuer- oder Regelvorrichtung (150);
- Betreiben wenigstens einer Blutpumpe (101) bei der eingestellten Pumprate (P1) während einer Förderdauer (T) derart, dass Fluid aus der Quelle (200) durch die erste Leitung (301) in die Aufnahme (400) gefördert wird;
- Ermitteln eines während der Förderdauer (T) geförderten Istvolumens (VI) und/oder einer tatsächlichen Pumprate mittels der ersten Wiegeeinrichtung (141);
- Ermitteln einer mathematischen Beziehung zwischen dem Istvolumen (VI) und einem sich aus der Förderdauer (T) und der eingestellten Pumprate (P1) ergebenden Sollvolumen (VS);
- Erzeugen von gleichen Druckverhältnissen vor und nach der Förderdauer (T) in der ersten Leitung (301).

13. Blutbehandlungsvorrichtung nach Anspruch 12, ausgestaltet als Hämodialysevorrichtung, Hämofiltrationsvorrichtung oder Hämodiafiltrationsvorrichtung, insbesondere als eine Vorrichtung für die chronische Nierenersatztherapie oder für die kontinuierliche Nierenersatztherapie (CRRT = continuous renal replacement therapy).

## Claims

1. A control or regulating device (150), suitable and provided and/or designed and/or configured to carry out a method for determining an actual pump rate and/or for calibrating a blood pump (101) of a blood treatment apparatus (100) which is connected to an extracorporeal blood circuit (300) with a dialyzer (303) having a semi-permeable membrane (303c), comprising the steps of:
- establishing a signal connection to the blood pump (101) which comprises or can be connected or is connected to:
- at least one first source (200) for a fluid;
- at least one first line (301) of an extracorporeal blood circuit (300), which connects downstream to the first source (200);
- at least one receptacle (400) for receiving fluid from the first source (200), the receptacle (400) being in fluid communication with the first line (301);
- wherein the receptacle (400) is arranged on or at a first weighing device (141) such that the weight of the receptacle (400) and/or its content can be determined by means of the weighing device (141);
wherein the method comprises the further steps of:
- setting a value for a pump rate adjustable at the blood pump (101) as a set pump rate (P1), or transmitting such a value to the blood pump (101) as a set pump rate (P1);
- operating the blood pump (101) at the set pump rate (P1) during a pumping time (T) such that fluid from the first source (200) is delivered as an actual volume (VI) through the first line (301) into the receptacle (400);
- determining an actual volume (VI) delivered during the pumping time (T) by means of the first weighing device (141) based on a change in the weight of the receptacle (400) or its content;
- determining a mathematical relationship between the actual volume (VI) and a target volume (VS) resulting from the pumping time (T) and the set pump rate (P1);
- generating equal pressure conditions before and after the pumping time (T) in the first line (301).

2. The control or regulating device (150) according to claim 1, wherein the method comprises the step of:
- determining a ratio between the set pump rate (P1) and an actual pump rate, the actual pump rate being determined by considering the actual volume (VI) and the pumping time (T).

3. The control or regulating device (150) according to claim 1 or 2, wherein the method comprises the step of:
- determining a correction factor or calibration factor for the blood pump (101) based on the ratio between the pump rate (P1) set at the blood pump (101) and the actual pump rate and/or based on the actual volume (VI) and the target volume (VS).

4. The control or regulating device (150) according to any one of the preceding claims, wherein the method comprises the step of:
- generating equal pressure conditions during the pumping time (T) in the first line (301).

5. The control or regulating device (150) according to any one of the preceding claims, wherein the receptacle (400) is in fluid communication with the first line (301) downstream of the dialyzer (303).

6. The control or regulating device (150) according to any one of the preceding claims, wherein the receptacle (400) is in fluid communication with the first line (301) downstream of the dialyzer (303) and through the dialyzer' s membrane (303c),

7. The control or regulating device (150) according to any one of the preceding claims, wherein the dialyzer (303) is connected to a dialysis fluid supply line (104) and a dialysate line (102), wherein a non-return valve (104a) and/or an occluding pump is/are integrated into the dialysis fluid supply line (104) to prevent an undesired flow, in particular in the direction opposite to the flow direction of the dialysis fluid supply line (104) and/or in the direction away from the dialyzer (303).

8. The control or regulating device (150) according to any one of the preceding claims, wherein the dialyzer (303) is in fluid communication with an arterial patient line (301) and with a venous patient line (305), wherein a tube clamp (306) is arranged in or on the venous patient line (305), wherein the method further comprises closing the tube clamp (306) on the venous patient line (305) during the pumping time (T).

9. The control or regulating device (150) according to any one of the preceding claims, wherein the blood pump (101) and an effluent pump (131) deliver at the same pump rate during the pumping time (T).

10. The control or regulating device (150) according to claim 10, wherein, to generate equal pressure conditions, the effluent pump (131) is rotated by the control or regulating device (150), in particular in a pressure-controlled manner.

11. The control or regulating device (150) according to any one of the preceding claims, with the step of:
- providing the blood treatment apparatus (100) as a hemodialysis apparatus, hemofiltration apparatus or hemodiafiltration apparatus, in particular designed as an apparatus for chronic renal replacement therapy or for continuous renal replacement therapy (CRRT).

12. A blood treatment apparatus (100) comprising or connected to an extracorporeal blood circuit (300), wherein the blood treatment apparatus (100) further comprises or is connected to:
- a blood pump (101) which is connectable to or connected to:
- at least one first source (200) for a fluid;
- at least one first line (301) which connects downstream to the first source (200);
- at least one receptacle (400) for receiving fluid from the first source (200), the receptacle (400) being in fluid communication with the first line (301);
- a first weighing device (141) for the receptacle (400);
a control or regulating device (150) according to any one of the claims 1-11 and/or configurated to:
- set a value for a pump rate adjustable at the blood pump (101) as a set pump rate (P1) by the user or automatically by the control or regulating device (150);
- operate at least one blood pump (101) at the set pump rate (P1) during a pumping time (T) such that fluid from the source (200) is delivered through the first line (301) into the receptacle (400);
- determine the actual volume (VI) delivered during the pumping time (T) and/or an actual pump rate by means of the first weighing device (141);
- determine a mathematical relationship between the actual volume (VI) and a target volume (VS) resulting from the pumping time (T) and the set pump rate (P1);
- generate equal pressure conditions before and after the pumping time (T) in the first line (301).

13. The blood treatment apparatus according to claim 12, designed as a hemodialysis apparatus, hemofiltration apparatus or hemodiafiltration apparatus, in particular as an apparatus for chronic renal replacement therapy or for continuous renal replacement therapy (CRRT).

## Revendications

1. Un dispositif de commande ou de régulation (150), adapté et prévu et/ou conçu et/ou configuré pour mettre en œuvre d'un procédé permettant de déterminer un débit de pompage réel et/ou de calibrer une pompe à sang (101) d'un appareil de traitement du sang (100) relié à un circuit sanguin extracorporel (300) ayant un dialyseur (303) muni d'une membrane semi-perméable (303c), comprenant les étapes suivantes :
- établir une liaison de signal avec la pompe à sang (101), qui comprend ou peut être reliée ou est reliée à :
- au moins une première source (200) pour un fluide;
- au moins un premier conduit (301) d'un circuit sanguin extracorporel (300), qui se raccorde en aval à la première source (200);
- au moins un réceptacle (400) destiné à recevoir le fluide de la première source (200), le réceptacle (400) étant en communication fluidique avec le premier conduit (301);
- où le réceptacle (400) est agencé sur ou contre un premier dispositif de pesage (141), de telle sorte que le poids du réceptacle (400) et/ou de son contenu puisse être déterminé au moyen du dispositif de pesage (141);
le procédé comprenant les étapes supplémentaires suivantes :
- définir une valeur pour un débit de pompage réglable à la pompe à sang (101) en tant que débit de pompage réglé (P1), ou transmettre une telle valeur à la pompe à sang (101) en tant que débit de pompage réglé (P1);
- actionner la pompe à sang (101) au débit de pompage réglé (P1) pendant une durée de pompage (T), de telle sorte que le fluide de la première source (200) soit acheminé en tant que volume réel (VI) au travers du premier conduit (301) dans le réceptacle (400);
- déterminer un volume réel (VI) acheminé pendant la durée de pompage (T) au moyen du premier dispositif de pesage (141) en fonction d'un changement de poids du réceptacle (400) ou de son contenu;
- déterminer une relation mathématique entre le volume réel (VI) et un volume cible (VS) résultant de la durée de pompage (T) et du débit de pompage réglé (P1);
- générer des conditions de pression identiques avant et après la durée de pompage (T) dans le premier conduit (301).

2. Le dispositif de commande ou de régulation (150) selon la première revendication, où le procédé comprend l'étape consistant à :
- déterminer un rapport entre le débit de pompage réglé (P1) et un débit de pompage réel, le débit de pompage réel étant déterminé en tenant compte du volume réel (VI) et de la durée de pompage (T).

3. Le dispositif de commande ou de régulation (150) selon la revendication 1 ou 2, où le procédé comprend l'étape consistant à :
- déterminer un facteur de correction ou un facteur de calibrage pour la pompe à sang (101) basé sur le rapport entre le débit de pompage (P1) réglé à la pompe à sang (101) et le débit de pompage réel et/ou basé sur le volume réel (VI) et le volume cible (VS).

4. Le dispositif de commande ou de régulation (150) selon l'une quelconque des revendications précédentes, où le procédé comprend l'étape consistant à :
- générer des conditions de pression identiques pendant la durée de pompage (T) dans le premier conduit (301).

5. Le dispositif de commande ou de régulation (150) selon l'une quelconque des revendications précédentes, où le réceptacle (400) est en communication fluidique avec le premier conduit (301) en aval du dialyseur (303).

6. Le dispositif de commande ou de régulation (150) selon l'une quelconque des revendications précédentes, où le réceptacle (400) est en communication fluidique avec le premier conduit (301) en aval du dialyseur (303) et au travers de la membrane (303c) du dialyseur.

7. Le dispositif de commande ou de régulation (150) selon l'une quelconque des revendications précédentes, où le dialyseur (303) est relié à un conduit d'alimentation en liquide de dialyse (104) ainsi qu'à un conduit de dialysat (102), où un clapet anti-retour (104a) ainsi qu'une pompe occlusive est/sont intégré(s) dans le conduit d'alimentation en liquide de dialyse (104) pour empêcher un flux indésirable, notamment dans le sens opposé à la direction d'écoulement du conduit d'alimentation en liquide de dialyse (104) et/ou dans le sens éloigné du dialyseur (303).

8. Le dispositif de commande ou de régulation (150) selon l'une quelconque des revendications précédentes, où le dialyseur (303) est en communication fluidique avec un conduit artériel du patient (301) ainsi qu'avec un conduit veineux du patient (305), où une pince de tuyau (306) est agencée dans ou sur le conduit veineux du patient (305), où le procédé comprend en outre une fermeture de la pince de tuyau (306) sur le conduit veineux du patient (305) pendant la durée de pompage (T).

9. Le dispositif de commande ou de régulation (150) selon l'une quelconque des revendications précédentes où la pompe à sang (101) et une pompe d'effluent (131) acheminent au même débit de pompage pendant la durée de pompage (T).

10. Le dispositif de commande ou de régulation (150) selon la revendication 10, où, pour générer des conditions de pression identiques, la pompe d'effluent (131) est entraînée par le dispositif de commande ou de régulation (150), notamment par régulation de pression.

11. Le dispositif de commande ou de régulation (150) selon l'une quelconque des revendications précédentes, comprenant l'étape consistant à :
- fournir l'appareil de traitement du sang (100) en tant qu'appareil d'hémodialyse, appareil d'hémofiltration ou appareil d'hémodiafiltration, conçu notamment comme un appareil pour la thérapie de remplacement rénal chronique ou pour la thérapie de remplacement rénal continue (CRRT = continuous renal replacement therapy).

12. Un appareil de traitement du sang (100) comprenant ou étant relié à un circuit sanguin extracorporel (300), où l'appareil de traitement du sang (100) comprend en outre ou est relié à :
- une pompe à sang (101) qui peut être raccordée ou est raccordée à :
- au moins une première source (200) pour un fluide;
- au moins un premier conduit (301) qui se raccorde en aval à la première source (200);
- au moins un réceptacle (400) destiné à recevoir le fluide de la première source (200), le réceptacle (400) étant en communication fluidique avec le premier conduit (301);
- un premier dispositif de pesage (141) pour le réceptacle (400);
un dispositif de commande ou de régulation (150) selon l'une quelconque des revendications 1 à 11 et/ou configuré pour :
- définir une valeur pour un débit de pompage réglable à la pompe à sang (101) en tant que débit de pompage réglé (P1), soit par l'utilisateur, soit automatiquement par le dispositif de commande ou de régulation (150);
- actionner au moins une pompe à sang (101) au débit de pompage réglé (P1) pendant une durée de pompage (T), de telle sorte que le fluide de la première source (200) soit acheminé au travers du premier conduit (301) dans le réceptacle (400);
- déterminer le volume réel (VI) acheminé pendant la durée de pompage (T) et/ou un débit de pompage réel au moyen du premier dispositif de pesage (141);
- déterminer une relation mathématique entre le volume réel (VI) et un volume cible (VS) résultant de la durée de pompage (T) et du débit de pompage réglé (P1);
- générer des conditions de pression identiques avant et après la durée de pompage (T) dans le premier conduit (301).

13. L'appareil de traitement du sang selon la revendication 12, conçu comme un appareil d'hémodialyse, un appareil d'hémofiltration ou un appareil d'hémodiafiltration, notamment, comme un appareil pour la thérapie de remplacement rénal chronique ou pour la thérapie de remplacement rénal continue (CRRT = continuous renal replacement therapy).
